# FASCICULE DE BREVET EUROPEEN

(11) **EP 0 852 134 B1**
(45) Date de publication et mention de la délivrance du brevet: **05.12.2001**
(21) Numéro de dépôt: 97402952.2
(22) Date de dépôt: 05.12.1997
(51) Int. Cl.: A61K 7/00, A61K 7/06, A61K 7/42

(54) **Utilisation d'une composition comprenant un céramide et un filtre UV sulfonique**
Verwendung von Zusammensetzungen, die ein Ceramid und einen UV-Filter mit Sulfonsäuregruppen enthalten
Use of a composition containing a ceramide and a sulphonic UV filter

(30) Priorité: 20.12.1996 FR 9615762
(43) Date de publication de la demande: 08.07.1998
(73) Titulaire: L'OREAL, 75008 Paris (FR)
(72) Inventeur: Dubief, Claude, 78150 Le Chesnay (FR); Cauwet-Martin, Danièle, 75011 Paris (FR)
(74) Mandataire: Andral, Christophe André Louis

(56) Documents cités:
- EP-A- 0 729 744
- WO-A-95/00111
- FR-A- 2 718 960
- STN, Serveur de Bases de Données, XP002043602

## Description

La présente invention a pour objet l'utilisation de composés de type céramide dans, ou pour la fabrication d'une composition capillaire comprenant au moins un filtre UV comportant au moins un radical acide sulfonique, destinée à protéger les cheveux.

On sait depuis longtemps que la lumière en particulier les ultraviolets dégradent les propriétés cosmétiques et/ou mécaniques des cheveux. Les cheveux sont alors ternes, rêches et cassants. Les cheveux, contrairement à la peau, éclaircissent.

On recherche depuis de nombreuses années, dans l'industrie cosmétique des substances permettant de protéger les cheveux des dégradations engendrées par les agressions atmosphériques, telles que la lumière et la chaleur.

Dans cette optique, on a déjà proposé d'utiliser les filtres solaires utilisés pour la photoprotection de la peau. Cependant, la structure de la peau et des cheveux sont très différentes et la demanderesse a ainsi constaté que la plupart des filtres utilisés dans les compositions pour la peau n'étaient pas efficaces pour la protection des cheveux.

La demanderesse a déjà proposé d'utiliser les filtres UV possédant un groupement sulfoniques pour la protection des propriétés mécaniques des cheveux dans les brevets français n° 2627085.
Cependant, ces filtres ne sont efficaces qu'à des concentrations importantes. Or, à ces concentrations, les cheveux traités avec ces filtres présentent un toucher rêche et chargé. De plus, le démêlage est extrêmement difficile.

La présente invention a donc pour but de proposer des compositions permettant de protéger efficacement les cheveux contre les agressions des rayons UV tout en apportant de bonnes propriétés de douceur et de démêlage des cheveux.

La demanderesse a trouvé de façon surprenante et inattendue que l'association d'un composé de type céramide dans une composition comprenant un filtre à groupement sulfonique permettait d'augmenter la quantité de filtre déposée sur les cheveux et par la même d'augmenter la protection. Cette augmentation du dépôt n'entraîne pas une diminution des propriétés de douceur et de démêlage. Au contraire, les cheveux présentent de très bonnes propriétés de douceur et de démêlage.

La présente invention a pour objet une composition cosmétique capillaire comprenant au moins un composé de type céramide et au moins un filtre UV comportant au moins un radical acide sulfonique ;
les composés de type céramide répondant à la formule générale (IX) suivante : 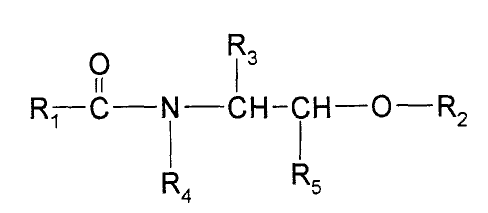 dans laquelle :
- R₁ désigne :
   - soit un radical hydrocarboné, linéaire ou ramifié, saturé ou insaturé, en C₁-C₅₀, de préférence en C₅-C₅₀, ce radical pouvant être substitué par un ou plusieurs groupements hydroxyle éventuellement estérifié par un acide R₇COOH, R₇ étant un radical hydrocarboné, saturé ou insaturé, linéaire ou ramifié, éventuellement mono ou polyhydroxylé, en C₁-C₃₅, le ou les hydroxyles du radical R₇ pouvant être estérifié par un acide gras saturé ou insaturé, linéaire ou ramifié, éventuellement mono ou polyhydroxylé, en C₁-C₃₅;
   - soit un radical R"-(NR-CO)-R', R désigne un atome d'hydrogène ou un radical hydrocarboné C₁-C₂₀ mono ou polyhydroxylé, préférentiellement monohydroxylé, R' et R" sont des radicaux hydrocarbonés dont la somme des atomes de carbone est comprise entre 9 et 30, R' étant un radical divalent.
   - soit un radical R₈-O-CO-(CH2)ₚ, R₈ désigne un radical hydrocarboné en C₁-C₂₀, p est un entier variant de 1 à 12.
- R₂ est choisi parmi un atome d'hydrogène, un radical de type saccharidique, en particulier un radical (glycosyle)ₙ, (galactosyle)ₘ ou sulfogalactosyle, un résidu de sulfate ou de phosphate, un radical phosphoryléthylamine et un radical phosphoryléthylammonium, dans lesquels n est un entier variant de 1 à 4 et m est un entier variant de 1 à 8 ;
- R₃ désigne un atome d'hydrogène ou un radical hydrocarboné en C₁-C₃₃, saturé ou insaturé, hydroxylé ou non, le ou les hydroxyles pouvant être estérifiés par un acide minéral ou un acide R₇COOH, R₇ ayant les mêmes significations que ci-dessus, le ou les hydroxyles pouvant être éthérifiés par un radical (glycosyle)ₙ, (galactosyle)ₘ, sulfogalactosyle, phosphoryléthylamine ou phosphoryléthylammonium, R₃ pouvant également être substitué par un ou plusieurs radicaux alkyle en C₁-C₁₄ ;
   de préférence, R₃ désigne un radical α-hydroxyalkyle en C₁₅-C₂₆, le groupement hydroxyle étant éventuellement estérifié par un α-hydroxyacide en C₁₆-C₃₀ ;
- R₄ désigne un atome d'hydrogène, un radical méthyle, éthyle, un radical hydrocarboné en C₃-C₅₀, saturé ou insaturé, linéaire ou ramifié, éventuellement hydroxylé ou un radical -CH₂-CHOH-CH₂-O-R₆ dans lequel R₆ désigne un radical hydrocarboné en C₁₀-C₂₆ ou un radical R₈-O-CO-(CH₂)ₚ, R₈ désigne un radical hydrocarboné en C₁-C₂₀, p est un entier variant de 1 à 12,
- R₅ désigne un atome d'hydrogène ou un radical hydrocarboné en C₁-C₃₀ saturé ou insaturé, linéaire ou ramifié, éventuellement mono ou polyhydroxylé, le ou les hydroxyles pouvant être éthérifiés par un radical (glycosyle)ₙ, (galactosyle)ₘ, sulfogalactosyle, phosphoryléthylamine ou phosphoryléthylammonium,
sous réserve que lorsque R₃ et R₅ désignent hydrogène ou lorsque R₃ désigne hydrogène et R₅ désigne méthyle alors R₄ ne désigne pas un atome d'hydrogène, un radical méthyle ou éthyle.

La présente invention a pour objet l'utilisation de composés de type céramide dans, ou pour la fabrication d'une composition capillaire comprenant au moins un filtre UV comportant au moins un radical acide sulfonique, destinée à protéger les cheveux.

La présente invention a pour objet l'utilisation d'un composé de type céramide dans une composition cosmétique capillaire comprenant un filtre UV comportant au moins un radical acide sulfonique pour améliorer le dépôt et/ou la fixation dudit filtre UV sur les cheveux.

Par utilisation capillaire, on entend selon la présente invention l'application de la composition sur les cheveux pour leur lavage et /ou leur traitement.

Selon l'invention, les filtres UV comportant au moins un radical acide sulfonique peuvent être notamment les dérivés sulfoniques du 3-benzylidène 2-camphre et notamment ceux de formules (I), (II), (III), (IV), et (V) suivantes : 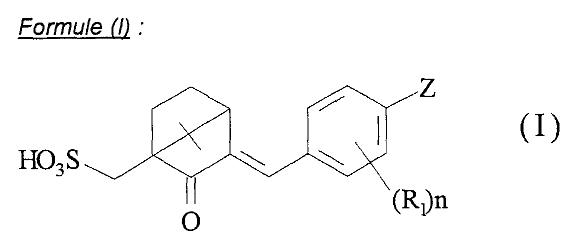 dans laquelle :
- Z désigne un groupement 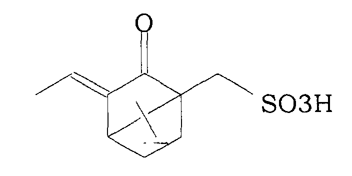
- n est égal à 0 ou est un nombre entier compris entre 1 et 4 (0 ≤ n ≤ 4),
- R₁ représente un ou plusieurs radicaux alkyle ou alkoxy, identiques ou différents, linéaires ou ramifiés, contenant de 1 à 4 atomes de carbone environ.

Un composé de formule (I) particulièrement préféré est celui correspondant à n = 0, à savoir l'acide benzène 1,4 [di(3-méthylidènecampho 10-sulfonique)]. 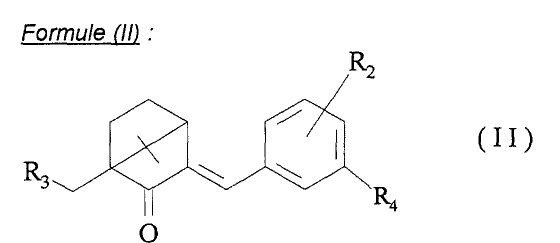 dans laquelle :
- R₂ désigne un atome d'hydrogène, un atome d'halogène, un radical alkyle contenant de 1 à 4 atomes de carbone environ ou un radical -SO₃H,
- R₃ et R₄ désignent un atome d'hydrogène ou un radical -SO₃H, l'un au moins des radicaux R₂, R₃ ou R₄ désignant le radical -SO₃H.

On peut citer, comme exemples particuliers, les composés suivants de formule (II) dans laquelle :
- R₂ désigne le radical -SO₃H en position para du benzylidènecamphre et R₃ et R₄ désignent chacun un atome d'hydrogène, c'est-à-dire l'acide 4-(3-méthylidènecamphre) benzène sulfonique.
- R₂ et R₄ désignent chacun un atome d'hydrogène et R₃ désigne un radical - SO₃H, c'est-à-dire l'acide 3-benzylidène campho-10-sulfonique.
- R₂ désigne un radical méthyle en position para du benzylidènecamphre, R₄ un radical -SO₃H et R₃ un atome d'hydrogène, c'est-à-dire l'acide 2-méthyl 5-(3-méthylidènecamphre) benzène sulfonique.
- R₂ désigne un atome de chlore en position para du benzylidènecamphre, R₄ un radical -SO₃H et R₃ un atome d'hydrogène, c'est-à-dire l'acide 2-chloro 5-(3-méthylidènecamphre) benzène sulfonique.
- R₂ désigne un radical méthyle en position para du benzylidènecamphre, R₄ désigne un atome d'hydrogène et R₃ désigne un radical -SO₃H, c'est-à-dire l'acide 3-(4-méthyl) benzylidène campho 10-sulfonique. 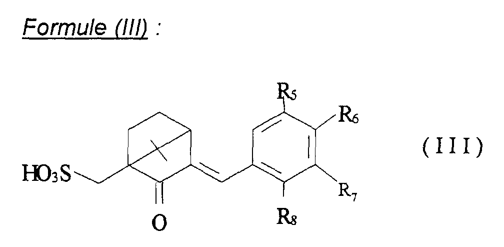
dans laquelle :
- R₅ et R₇ désignent un atome d'hydrogène, un radical hydroxyle, un radical alkyle ou alcoxy, linéaire ou ramifié, contenant de 1 à 8 atomes de carbone environ, l'un au moins des radicaux R₅ et R₇ représentant un radical hydroxyle, alkyle ou alcoxy,
- R₆ et R₈ désignent un atome d'hydrogène, un radical hydroxyle, l'un au moins des radicaux R₆ et R₈ désignant le radical hydroxyle.

On peut citer, comme exemples particuliers, les composés suivants de formule (III) dans laquelle :
- R₅ est un radical méthyle, R₆ un atome d'hydrogène, R₇ un radical tertiobutyle, R₈ un radical hydroxyle, c'est-à-dire l'acide (3-t-butyl 2-hydroxy 5-méthyl) benzylidène campho-10-sulfonique.
- R₅ est un radical méthoxy, R₆ un atome d'hydrogène, R₇ un radical tertiobutyle, R₈ un radical hydroxyle, c'est-à-dire l'acide (3-t-butyl 2-hydroxy 5-méthoxy) benzylidène campho-10-sulfonique.
- R₅ et R₇ désignent chacun un radical tertiobutyle, R₆ un radical hydroxyle, R₈ un atome d'hydrogène, c'est-à-dire l'acide (3,5-diterbutyl 4-hydroxy) benzylidène campho-10-sulfonique. 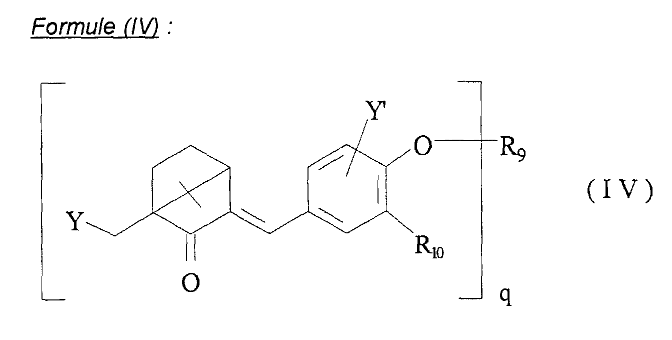
dans laquelle :
- R₉ désigne un atome d'hydrogène, un radical alkyle, linéaire ou ramifié, contenant de 1 à 18 atomes de carbone environ, un radical alcényle, linéaire ou ramifié, contenant de 3 à 18 atomes de carbone environ, un groupement 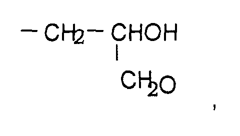 ou -(CH₂CH₂O)ₙ-H, ou CH₂-CHOH-CH₃ ou encore un radical divalent : -(CH₂)ₘ- ou -CH₂ -CHOH-CH₂-,
   n étant un nombre entier compris entre 1 et 6 (1 ≤ n ≤ 6) et m un nombre entier compris entre 1 et 10 (1 ≤ m ≤ 10),
- R₁₀ désigne un atome d'hydrogène, un radical alcoxy contenant de 1 à 4 atomes de carbone environ ou un radical divalent - O - relié au radical R₉ lorsque celui-ci est divalent lui aussi,
- q désigne un nombre entier égal à 1 ou 2, étant entendu que si q est égal à 2, R₉ doit désigner un radical divalent,
- Y et Y' désignent un atome d'hydrogène ou un radical -SO₃H, au moins un de ces radicaux Y ou Y' étant différent de l'hydrogène.

On peut citer, comme exemples particuliers, les composés suivants de formule (IV) dans laquelle :
- q est égal à 1, Y et R₁₀ désignent chacun un atome d'hydrogène, R₉ désigne un radical méthyle, Y' en position 3 désigne un radical -SO₃H, c'est-à-dire l'acide 2-méthoxy 5-(3-méthylidènecamphre) benzène sulfonique.
- q est égal à 1, Y désigne un radical -SO₃H, Y' un atome d'hydrogène, R₁₀ un radical divalent -O- relié à R₉ désignant un radical méthylène, c'est-à-dire l'acide 3-(4,5-méthylènedioxy) benzylidène campho-10-sulfonique.
- q est égal à 1, Y désigne un radical -SO₃H, Y' et R₁₀ désignent tous deux un atome d'hydrogène, R₉ désigne un radical méthyle, c'est-à-dire l'acide 3-(4-méthoxy) benzylidène campho-10-sulfonique.
- q est égal à 1, Y désigne un radical -SO₃H, Y' un atome d'hydrogène, R₉ désigne un radical méthyle, R₁₀ désigne un radical méthoxy, c'est-à-dire l'acide 3-(4,5-diméthoxy) benzylidène campho-10-sulfonique.
- q est égal à 1, Y désigne un radical -SO₃H, Y' et R₁₀ désignent tous deux un atome d'hydrogène, et R₉ un radical n butyle, c'est-à-dire l'acide 3-(4-n-butoxy) benzylidène campho-10-sulfonique.
- q est égal à 1, Y désigne un radical -SO₃H, Y' un atome d'hydrogène, R₉ désigne un radical n butyle, R₁₀ un radical méthoxy, c'est-à-dire l'acide 3-(4-n-butoxy 5-méthoxy) benzylidène campho-10-sulfonique. 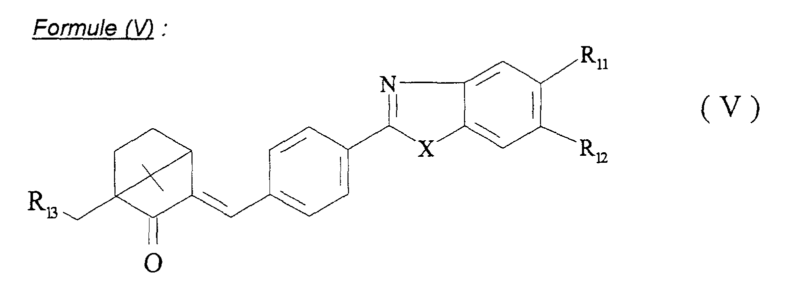
dans laquelle :
- R₁₁ désigne un atome d'hydrogène, un radical alkyle ou alcoxy, linéaire ou ramifié, contenant de 1 à 6 atomes de carbone environ ou un radical -SO₃H,
- R₁₂ désigne un atome d'hydrogène, un radical alkyle ou alcoxy, linéaire ou ramifié, contenant de 1 à 6 atomes de carbone environ,
- R₁₃ désigne un atome d'hydrogène ou un radical -SO₃H, l'un au moins des radicaux R₁₁ et R₁₃ désignant un radical -SO₃H,
- X est un atome d'oxygène ou de soufre ou un groupement -NR-, R étant un atome d'hydrogène ou un radical alkyle, linéaire ou ramifié, contenant de 1 à 6 atomes de carbone environ.

On peut citer, comme exemple particulier de formule (V), le composé dans lequel X désigne un radical -NH-, R₁₁ désigne un radical -SO₃H, R₁₂ et R₁₃ désignent tous deux un atome d'hydrogène, c'est-à-dire l'acide 2-[4-(camphométhylidène) phényl] benzimidazole-5-sulfonique.

Les composés de formules (I), (II), (III), (IV), (V) ci-dessus sont respectivement décrits dans le brevet US 4 585 597 et les demandes de brevets FR 2 236 515, 2 282 426, 2 645 148, 2 430 938 et 2 592 380.

Le filtre à groupement sulfonique peut également être un dérivé sulfonique de benzophénone de formule (VI) suivante : 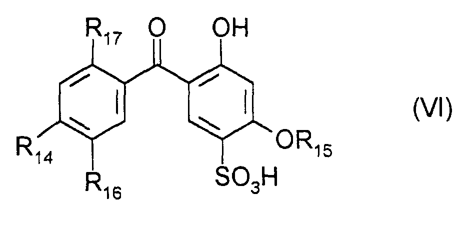 dans laquelle :
- R₁₄ désigne soit un atome d'hydrogène soit un radical alkoxy, linéaire ou ramifié, contenant de 1 à 8 atomes de carbone,
- R₁₅ désigne soit un atome d'hydrogène soit un radical alkyle, linéaire ou ramifié, contenant de 1 à 8 atomes de carbone,
- R₁₆ désigne soit un atome d'hydrogène, soit un groupement SO₃H
- R₁₇ désigne soit un atome d'hydrogène soit un radical hydroxyle.

On peut citer comme exemple particulier de composé de formule (VI) : l'acide 2-hydroxy 4-méthoxybenzophénone 5-sulfonique (composé de formule (VI) dans laquelle a, b, et c sont égaux à zéro, et R₁₅ désigne un radical méthyle).

Le filtre à groupement sulfonique peut encore être un dérivé sulfonique de formule (VII) suivante : 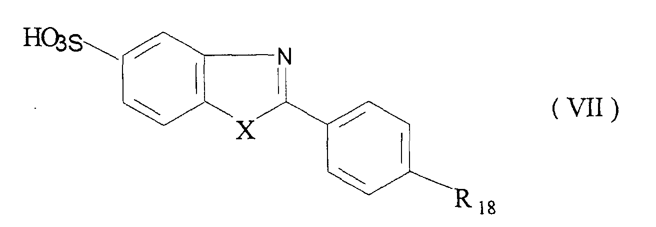 dans laquelle :
- X désigne un atome d'oxygène ou un radical - NH -,
- R₁₈ désigne un atome d'hydrogène, un radical alkyle ou alcoxy, linéaire ou ramifié, contenant de 1 à 8 atomes de carbone ou un groupement de formule (VIII) 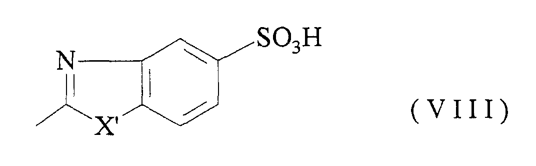
dans laquelle X' représente un atome d'oxygène ou un radical -NH-.

On peut citer, comme exemples particuliers, les composés suivants de formule (VII) dans laquelle :
- X désigne le radical -NH- et R₁₆ désigne un atome d'hydrogène : l'acide 2-phénylbenzimidazole 5-sulfonique.
- X désigne le radical -NH-, R₁₆ désigne le groupement de formule (VIII) dans lequel X' désigne le radical -NH- : l'acide benzène 1,4 -di(benzimidazol -2 yl-5-sulfonique).
- X désigne un atome d'oxygène, R₁₆ désigne le groupement de formule (VIII) dans lequel X' désigne un atome d'oxygène : l'acide benzène 1,4-di (benzoxazol -2 yl -5-sulfonique).

Les composés de formule (VI) et (VII) sont des composés connus pouvant être préparés selon des méthodes classiques décrites dans l'art antérieur.

Des exemples de compositions cosmétiques filtrantes préférées dans le cadre de la présente invention comprennent les filtres UV hydrophiles acides suivants :
- dérivé sulfonique de 3-benzylidène 2-camphre de formule (I) dans laquelle
   n = 0 (acide benzène 1,4 (di(3-méthylidène campho-10-sulfonique))
- dérivé sulfonique de benzophénone de formule (IV) dans laquelle a, b et c sont égaux à 0, et R₁₅ désigne un radical méthyle (acide 2-hydroxy 4-méthoxybenzophénone 5- sulfonique, notamment vendu par BASF sous le nom de UVINUL MS 40).
- dérivé sulfonique de benzimidazole de formule (VII) dans laquelle X désigne le radical -NH- et R₁₆ désigne un atome d'hydrogène (acide 2-phénylbenzimidazole 5-sulfonique, notamment vendu par MERCK sous le nom de EUSOLEX 232).

Le filtre UV hydrophile comportant au moins un radical acide sulfonique, est généralement présent dans les compositions selon l'invention à une concentration totale comprise entre 0,05 et 10 % en poids environ, et de préférence entre 0,25 et 6 % en poids environ, par rapport au poids total de la composition.

Selon la présente invention, on entend, par composé de type céramide, les céramides et/ou les glycocéramides et/ou les pseudocéramides et/ou les néocéramides, naturelles ou synthétiques.
Des composés de type céramides sont par exemple décrits dans les demandes de brevet DE4424530, DE4424533, DE4402929, DE4420736, WO95/23807, WO94/07844, EP-A-0646572, WO95/16665, FR-2 673 179, EP-A-0227994 et WO 94/07844, WO94/24097, WO94/10131.
Parmi les composés de formule (I), on préfère les céramides et/ou glycocéramides dont la structure est décrite par DOWNING dans Journal of Lipid Research Vol. 35, 2060-2068, 1994, ou ceux décrits dans la demande de brevet français FR-2 673 179.

Les composés de type céramide plus particulièrement préférés selon l'invention sont les composés de formule (I) pour lesquels R₁ désigne un alkyle saturé ou insaturé dérivé d'acides gras en C₁₄-C₂₂ éventuellement hydroxylé; R₂ désigne un atome d'hydrogène ; et R₃ désigne un radical linéaire en C₁₁₋₁₇ éventuellement hydroxylé et de préférence en C₁₃₋₁₅.

De tels composés sont par exemple :
- le 2-N-linoléoylamino-octadécane-1,3-diol,
- le 2-N-oléoylamino-octadécane-1,3-diol,
- le 2-N-palmitoylamino-octadécane-1,3-diol,
- le 2-N-stéaroylamino-octadécane-1,3-diol,
- le 2-N-béhénoylamino-octadécane-1,3-diol,
- le 2-N-[2-hydroxy-palmitoyl]-amino-octadécane-1,3-diol,
- le 2-N-stéaroyl amino-octadécane-1,3,4 triol et en particulier la N-stéaroyl phytosphingosine,
- le 2-N-palmitoylamino-hexadécane-1,3-diol
ou les mélanges de ces composés.

On peut aussi utiliser des mélanges spécifiques tels que par exemple les mélanges de céramide(s) 2 et de céramide(s) 5 selon la classification de DOWNING.

On peut également utiliser les composés de formule (I) pour lesquels R₁ désigne un radical alkyle saturé ou insaturé dérivé d'acides gras en C₁₂-C₂₂ ; R₂ désigne un radical galactosyle ou sulfogalactosyle ; et R₃ désigne un radical hydrocarboné en C₁₂-C₂₂, saturé ou insaturé et de préférence un groupement -CH=CH-(CH₂)₁₂₋CH₃.

A titre d'exemple, on peut citer le produit constitué d'un mélange de glycocéramides, vendu sous la dénomination commerciale GLYCOCER par la société WAITAKI INTERNATIONAL BIOSCIENCES.

On peut également utiliser les composés de formule (I) décrits dans les demandes de brevet EP-A-0227994, EP-A-0 647 617, EP-A-0 736 522 et WO 94/07844.

De tels composés sont par exemple le QUESTAMIDE H (bis-(N-hydroxyéthyl N-cétyl) malonamide) vendu par la société QUEST, le N-(2-hydroxyéthyl)-N-(3-cétyloxy-2-hydroxypropyl)amide d'acide cétylique.

On peut également utiliser le N-docosanoyl N-méthyl-D-glucamine décrit dans la demande de brevet WO94/24097.

La concentration en composés de type céramide peut varier entre 0,0001% et 20% en poids environ par rapport au poids total de la composition, et de préférence entre 0,001 et 10% environ et encore plus préférentiellement entre 0,005 et 3 % en poids.

Les compositions selon l'invention peuvent se présenter sous forme de shampooing, de lotion, de gel, d'émulsion, de dispersion vésiculaire non ionique, de laque pour cheveux et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.
Ces compositions comprennent les ingrédients habituellement utilisés dans le domaine capillaire, et peuvent être préparées selon les méthodes usuelles connues de l'homme du métier.

Les compositions peuvent par ailleurs contenir des additifs cosmétiques conventionnels choisis parmi les corps gras, les solvants organiques, les silicones, les épaississants, les adoucissants, les tensioactifs, les polymères anioniques, cationiques, non-ioniques ou amphotères, les agents anti-mousses, les agents conditionneurs du cheveu tels que des protéines, des vitamines, les agents traitants (agents anti-chute, antipelliculaires), les colorants, les parfums, les conservateurs, les agents propulseurs.

Un autre objet de la présente invention est un procédé de traitement cosmétique des cheveux destiné à les protéger contre les effets des rayons UV consistant à appliquer sur ceux-ci une quantité efficace d'une composition cosmétique telle que définie ci-dessus.

Des exemples concrets, mais nullement limitatifs, illustrant l'invention, vont maintenant être donnés.

### EXEMPLE 1

On a préparé un shampooing de composition suivante :
- Acide 2-hydroxy 4-méthoxy benzophénone 5-sulfonique vendu par la société RHON POULENC sous la dénomination RHODIALUX S 0,5 gMA
- 2-N-oléoylamino-octadécane-1,3-diol 0,5 g
- Lauryléthersulfate de sodium à 2,2 moles d'oxyde d'éthylène en solution aqueuse à 28% de MA 13,8 gMA
- Cocoylbétaïne en solution aqueuse à 30% deMA (DEHYTON AB 30 de HENKEL) 2,5 gMA
- Eau qsp 100 g

Le pH est ajusté à 5 avec de la soude.

Ce shampooing protège les cheveux contre les UV et le démêlage des cheveux lavés avec cette composition est aisé.

### EXEMPLE 2

On a préparé un après-shampooing rincé de composition suivante :
- Acide benzène 1,4 (di(3-méthylidènecampho-10-sulfonique)] 1 gMA
- 2-N-oléoylamino-octadécane-1,3-diol 1 g
- Polyacrylamide (SEPIGEL 305 de SEPPIC) 2 gMA
- Mélange (13/87 en poids) de diméthiconol et de cyclométhicone (Q2-1401 de DOW CORNING) 10 g
- Conservateurs, parfum qs
- Eau qsp 100 g

Le pH est ajusté à 5 avec de la soude.

Cet après-shampooing est appliqué sur cheveux lavés et essorés. Après un temps de pause, les cheveux sont rincés à l'eau. La composition protège les cheveux contre les UV et les cheveux traités avec cette composition sont doux et faciles à démêler.

### EXEMPLE 3

On a préparé une lotion A de composition suivante :
- Acide benzène 1,4 (di(3-méthylidènecampho-10-sulfonique)] en solution aqueuse à 33% de MA 0,5 gMA
- 2-N-oléoylamino-octadécane-1,3-diol 0,5 g
- Cocoylbétaïne en solution aqueuse à 30% deMA (DEHYTON AB 30 de HENKEL) 3 gMA
- Conservateurs qs
- Eau qsp 100 g

Le pH est ajusté à 5 avec de la soude.

Cette lotion est appliquée sur des cheveux lavés et essorés. Après un temps de pause, les cheveux sont rincés à l'eau. La composition protège les cheveux contre les UV et les cheveux traités avec cette composition sont doux et faciles à démêler.

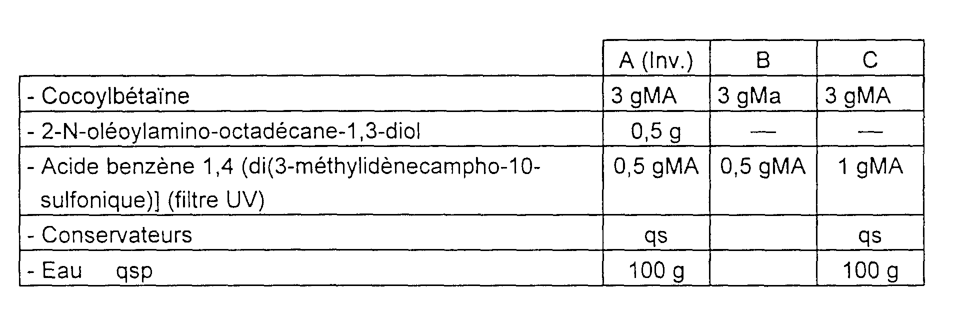

On a appliqué 1 g de chacune des compositions A, B, C sur des mèches de 2,5 g. On a laissé poser 10 mn puis on a effectué un rinçage sous l'eau du robinet.
On a ensuite demandé à un panel de 10 experts de juger le démêlage et la douceur des cheveux mouillés.

Les 10 experts à l'unanimité ont préféré la mèche traitée avec la composition A selon l'invention. Les cheveux traités avec la composition A se démêlent plus facilement et sont plus doux que ceux traités avec les compositions B ou C.

On a également comparé la quantité du filtre UV fixée sur les cheveux traités avec ces trois compositions.

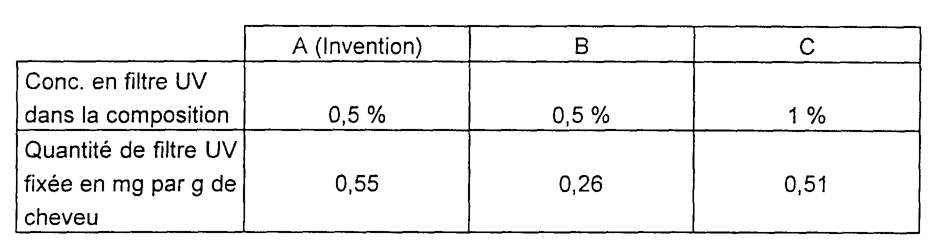

On constate que la quantité fixée lorsqu'on a appliqué la composition A selon l'invention est le double de celle fixée lorsqu'on a appliqué la composition B contenant la même concentration en filtre UV mais ne contenant pas de composé de type céramide.

## Revendications

1. Composition cosmétique capillaire, **caractérisée en ce qu'**elle comprend, dans un milieu cosmétiquement acceptable, au moins un composé de type céramide et au moins un agent filtrant le rayonnement ultraviolet, comportant au moins un radical acide sulfonique ;
le composé de type céramide répondant à la formule générale (IX): 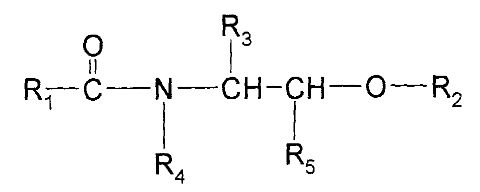 dans laquelle :
- R₁ désigne :
- soit un radical hydrocarboné, linéaire ou ramifié, saturé ou insaturé, en C₁-C₅₀, de préférence en C₅-C₅₀, ce radical pouvant être substitué par un ou plusieurs groupements hydroxyle éventuellement estérifié par un acide R₇COOH, R₇ étant un radical hydrocarboné, saturé ou insaturé, linéaire ou ramifié, éventuellement mono ou polyhydroxylé, en C₁-C₃₅, le ou les hydroxyles du radical R₇ pouvant être estérifié par un acide gras saturé ou insaturé, linéaire ou ramifié, éventuellement mono ou polyhydroxylé, en C₁-C₃₅;
- soit un radical R"-(NR-CO)-R', R désigne un atome d'hydrogène ou un radical hydrocarboné C₁-C₂₀ mono ou polyhydroxylé, préférentiellement monohydroxylé, R' et R" sont des radicaux hydrocarbonés dont la somme des atomes de carbone est comprise entre 9 et 30, R' étant un radical divalent.
- soit un radical R₈-O-CO-(CH2)ₚ, R₈ désigne un radical hydrocarboné en C₁-C₂₀, p est un entier variant de 1 à 12.
- R₂ est choisi parmi un atome d'hydrogène, un radical de type saccharidique, en particulier un radical (glycosyle)ₙ, (galactosyle)ₘ ou sulfogalactosyle, un résidu de sulfate ou de phosphate, un radical phosphoryléthylamine et un radical phosphoryléthylammonium, dans lesquels n est un entier variant de 1 à 4 et m est un entier variant de 1 à 8 ;
- R₃ désigne un atome d'hydrogène ou un radical hydrocarboné en C₁-C₃₃, saturé ou insaturé, hydroxylé ou non, le ou les hydroxyles pouvant être estérifiés par un acide minéral ou un acide R₇COOH, R₇ ayant les mêmes significations que ci-dessus, le ou les hydroxyles pouvant être éthérifiés par un radical (glycosyle)ₙ, (galactosyle)ₘ, sulfogalactosyle, phosphoryléthylamine ou phosphoryléthylammonium, R₃ pouvant également être substitué par un ou plusieurs radicaux alkyle en C₁-C₁₄ ;
de préférence, R₃ désigne un radical α-hydroxyalkyle en C₁₅-C₂₆, le groupement hydroxyle étant éventuellement estérifié par un α-hydroxyacide en C₁₆-C₃₀;
- R₄ désigne un atome d'hydrogène, un radical méthyle, éthyle, un radical hydrocarboné en C₃-C₅₀, saturé ou insaturé, linéaire ou ramifié, éventuellement hydroxylé ou un radical -CH₂-CHOH-CH₂-O-R₆ dans lequel R₆ désigne un radical hydrocarboné en C₁₀-C₂₆ ou un radical R₈-O-CO-(CH2)ₚ, R₈ désigne un radical hydrocarboné en C₁-C₂₀, p est un entier variant de 1 à 12,
- R₅ désigne un atome d'hydrogène ou un radical hydrocarboné en C₁-C₃₀ saturé ou insaturé, linéaire ou ramifié, éventuellement mono ou polyhydroxylé, le ou les hydroxyles pouvant être éthérifiés par un radical (glycosyle)ₙ, (galactosyle)ₘ sulfogalactosyle, phosphoryléthylamine ou phosphoryléthylammonium,
sous réserve que lorsque R₃ et R₅ désignent hydrogène ou lorsque R₃ désigne hydrogène et R₅ désigne méthyle alors R₄ ne désigne pas un atome d'hydrogène, un radical méthyle ou éthyle.

2. Composition selon la revendication 1, **caractérisée en ce que** l'agent filtrant le rayonnement ultraviolet est un composé de structure 3-benzylidène 2-camphre.

3. Composition selon la revendication 2, **caractérisé en ce que** l'agent filtrant le rayonnement ultraviolet répond à l'une des formules (I) à (V) suivantes : 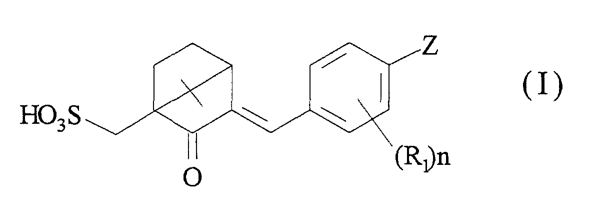 dans laquelle :
- Z désigne un groupement : 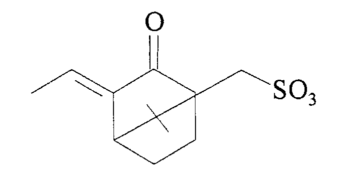
- n désigne 0 ou un nombre entier supérieur ou égal à 1 et inférieur ou égal à 4,
- R₁ représente un ou plusieurs radicaux alkyle ou alkoxy, identiques ou différents, linéaires ou ramifiés, contenant de 1 à 4 atomes de carbone. 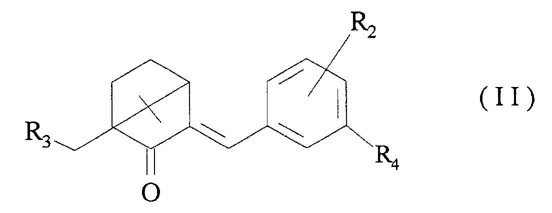
dans laquelle
- R₂ désigne un atome d'hydrogène, un atome d'halogène, un radical alkyle contenant 1 à 4 atomes de carbone, un radical -SO₃H,
- R₃ et R₄ désignent un atome d'hydrogène ou un radical -SO₃H, l'un au moins des radicaux R₂, R₃ ou R₄ désignant le radical -SO₃H. 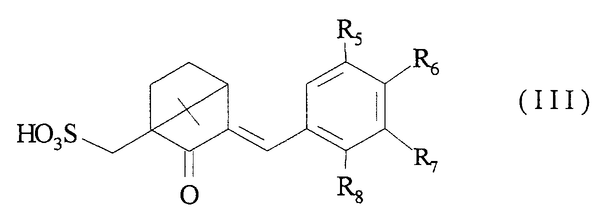
dans laquelle :
- R₅ et R₇, identiques ou différents, désignent un atome d'hydrogène, un radical hydroxyle, un radical alkyle linéaire ou ramifié contenant 1 à 8 atomes de carbone ou un radical alcoxy linéaire ou ramifié contenant 1 à 8 atomes de carbone, l'un au moins des radicaux R₅ et R₇ représentant un radical hydroxyle, alkyle ou alcoxy,
- R₆ et R₈, identiques ou différents, désignent un atome d'hydrogène, un radical hydroxyle, l'un au moins des radicaux R₆ et R₈ désignant le radical hydroxyle. 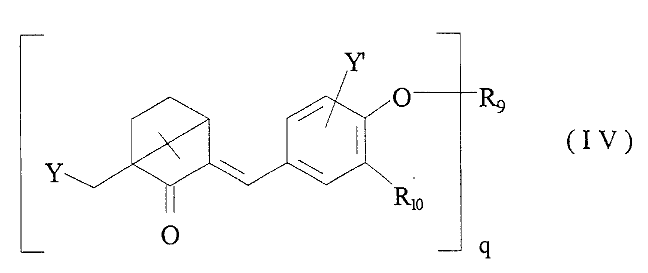
dans laquelle
- R₉ désigne un atome d'hydrogène, un radical alkyle linéaire ou ramifié contenant de 1 à 18 atomes de carbone , un radical alcényle, linéaire ou ramifié, contenant de 3 à 18 atomes de carbone, un groupement choisi parmi : 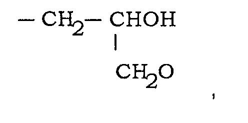 ou -(CH₂CH₂O)ₙ-H, ou CH₂-CHOH-CH₃, ou -(CH₂)ₘ- ou -CH₂ -CHOH-CH₂-, n étant un nombre entier compris entre 1 et 6 (1 ≤ n ≤ 6) et m un nombre entier compris entre 1 et 10 (1 ≤ m ≤ 10),
- R₁₀ désigne un atome d'hydrogène, un radical alcoxy contenant de 1 à 4 atomes de carbone, ou un radical divalent - O - relié au radical R₉ lorsque celui-ci est divalent lui aussi.
- q désigne un nombre entier égal à 1 ou 2, étant entendu que si q est égal à 2, R₉ doit désigner un radical divalent.
- Y et Y' désignent un atome d'hydrogène ou un radical -SO₃H, au moins un de ces radicaux Y ou Y' est différent de l'hydrogène. 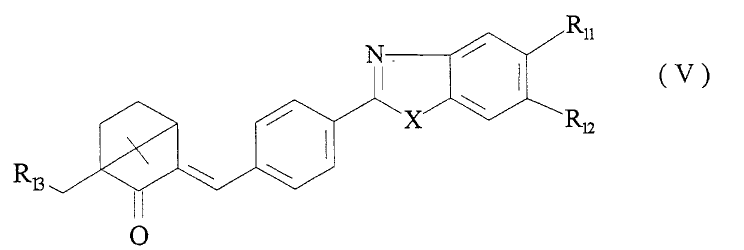
dans laquelle :
- R₁₁ désigne un atome d'hydrogène, un radical alkyle ou alcoxy, linéaire ou ramifié, contenant 1 à 6 atomes de carbone, un radical -SO₃H,
- R₁₂ désigne un atome d'hydrogène, un radical alkyle ou alcoxy linéaire ou ramifié contenant 1 à 6 atomes de carbone,
- R₁₃ désigne un atome d'hydrogène, ou un radical -SO₃H,
- l'un au moins des radicaux R₁₁ ou R₁₃ désigne un radical -SO₃H,
- X est un atome d'oxygène ou de soufre, ou un groupement -NR-, R étant un atome d'hydrogène ou un radical alkyle linéaire ou ramifié contenant de 1 à 6 atomes de carbone.

4. Composition selon la revendication 3, **caractérisée en ce que** le composé de formule (I) est l'acide benzène 1,4 [di(3-méthylidènecampho-10-sulfonique)], les composés de formule (II) sont choisis parmi l'acide 4-(3-méthylidènecamphre) benzène sulfonique, l'acide 3-benzylidène campho-10-sulfonique, l'acide 2-méthyl 5-(3-méthylidènecamphre) benzène sulfonique, l'acide 2-chloro 5-(3-méthylidènecamphre) benzène sulfonique, l'acide 3-(4-méthyl) benzylidène campho 10-sulfonique, les composés de formule (III) sont choisis parmi l'acide (3-t-butyl 2-hydroxy 5-méthyl) benzylidène campho-10-sulfonique, l'acide (3-t-butyl 2-hydroxy 5-méthoxy) benzylidène campho-10-sulfonique, l'acide (3,5-diterbutyl 4-hydroxy) benzylidène campho-10-sulfonique, les composés de formule (IV) sont choisis parmi l'acide 2-méthoxy 5-(3-méthylidènecamphre) benzène sulfonique, l'acide 3-(4,5-méthylènedioxy) benzylidène campho-10-sulfonique, l'acide 3-(4-méthoxy) benzylidène campho-10-sulfonique, l'acide 3-(4,5-diméthoxy) benzylidène campho-10-sulfonique, l'acide 3-(4-n-butoxy) benzylidène campho-10-sulfonique, l'acide 3-(4-n-butoxy 5-méthoxy) benzylidène campho-10-sulfonique, le composé de formule (V) est l'acide 2-[4-(camphométhylidène) phényl] benzimidazole-5-sulfonique.

5. Composition selon la revendication 1, **caractérisée en ce que** l'agent filtrant le rayonnement ultraviolet est un composé de formule (VI) : 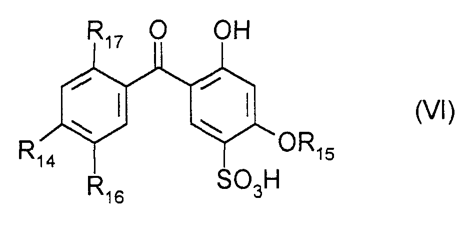 dans laquelle :
- R₁₄ désigne soit un atome d'hydrogène soit un radical alkoxy, linéaire ou ramifié, contenant de 1 à 8 atomes de carbone,
- R₁₅ désigne soit un atome d'hydrogène soit un radical alkyle, linéaire ou ramifié, contenant de 1 à 8 atomes de carbone,
- R₁₆ désigne soit un atome d'hydrogène, soit un groupement SO₃H
- R₁₇ désigne soit un atome d'hydrogène soit un radical hydroxyle.

6. Composition selon la revendication 5, **caractérisée en ce que** le composé de formule (VI) est l'acide 2-hydroxy 4-méthoxybenzophénone 5-sulfonique.

7. Composition selon la revendication 1, **caractérisée par le fait que** l'agent filtrant le rayonnement ultraviolet est un composé de formule (VII) : 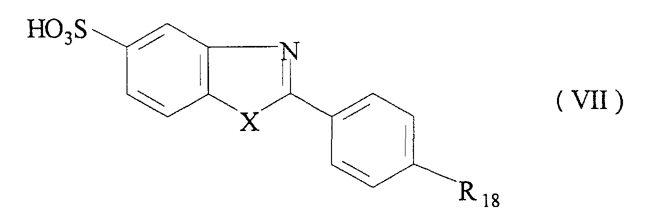 dans laquelle :
- X désigne un atome d'oxygène ou un radical - NH -,
- R18 désigne un atome d'hydrogène, un radical alkyle ou alcoxy, linéaire ou ramifié, contenant de 1 à 8 atomes de carbone ou un groupement de formule (VIII) 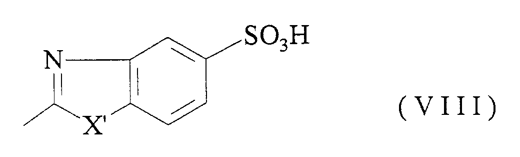
dans laquelle X' désigne indépendamment de X, un atome oxygène ou un radical - NH-.

8. Composition selon la revendication 7, **caractérisée en ce que** le composé de formule (VII) est choisi parmi : l'acide 2-phénylbenzimidazole 5-sulfonique, l'acide benzène 1,4 -di(benzimidazol -2 yl-5-sulfonique), l'acide benzène 1,4-di (benzoxazol - 2 yl -5-sulfonique).

9. Composition selon l'une quelconque des revendications 1 à 8, **caractérisée en ce que** le composé de type céramide est choisi dans le groupe constitué par :
- le 2-N-linoléoylamino-octadécane-1,3-diol,
- le 2-N-oléoylamino-octadécane-1,3-diol,
- le 2-N-palmitoylamino-octadécane-1,3-diol,
- le 2-N-stéaroylamino-octadécane-1,3-diol,
- le 2-N-béhénoylamino-octadécane-1,3-diol,
- le 2-N-[2-hydroxy-palmitoyl]-amino-octadécane-1,3-diol,
- le 2-N-stéaroyl amino-octadécane-1,3,4 triol,
- le 2-N-palmitoylamino-hexadécane-1,3-diol,
ou les mélanges de ces composés.

10. Composition selon l'une quelconque des revendications 1 à 8, **caractérisée en ce que** le composé de type céramide est choisi parmi le bis-(N-hydroxyéthyl N-cétyl) malonamide, le N-(2-hydroxyéthyl)-N-(3-cétyloxy-2-hydroxypropyl)amide d'acide cétylique) et le N-docosanoyl N-méthyl-D-glucamine.

11. Composition selon l'une quelconque des revendications précédentes, **caractérisée par le fait qu'**elle renferme de 0,05 à 10 % en poids d'agent filtrant le rayonnement ultraviolet, et de préférence 0,1 à 5 % en poids, par rapport au poids total de la composition.

12. Utilisation des compositions définies à l'une quelconque des revendications 1 à 11 comme, ou pour la fabrication de, compositions cosmétiques pour la protection des cheveux contre le rayonnement ultraviolet, en particulier le rayonnement solaire.

13. Procédé de traitement cosmétique pour protéger les cheveux contre le rayonnement ultraviolet, en particulier le rayonnement solaire, **caractérisé en ce qu'**il consiste à appliquer sur ceux-ci une quantité efficace d'une composition telle que définie à l'une quelconque des revendications 1 à 11.

14. Utilisation d'un composé de type céramide dans une composition cosmétique capillaire contenant un filtre UV hydrophile comportant au moins un radical acide sulfonique pour améliorer la fixation dudit filtre UV sur les cheveux.

## Patentansprüche

1. Kosmetische Zusammensetzung für das Haar, **dadurch gekennzeichnet, daß** sie in einem kosmetisch akzeptablen Medium mindestens eine Verbindung vom Ceramidtyp und mindestens ein UV-Filter, das mindestens eine Sulfonsäuregruppe aufweist, enthält,
wobei die Verbindungen vom Ceramidtyp der folgenden allgemeinen Formel (IX) entsprechen: 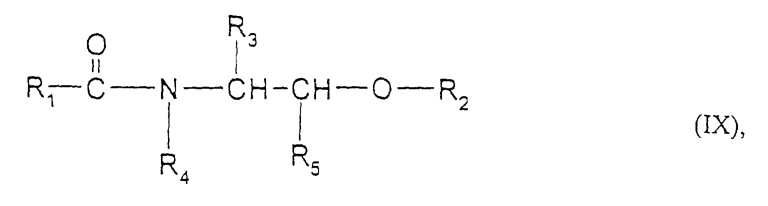 worin bedeuten:
- R₁
entweder eine geradkettige oder verzweigte, gesättigte oder ungesättigte C₁₋₅₀-Kohlenwasserstoffgruppe und vorzugsweise C₅₋₅₀-Kohlenwasserstoffgruppe, wobei die Gruppe mit einer oder mehreren Hydroxygruppen substituiert sein kann, die gegebenenfalls mit einer Säure R₇COOH verestert sein können, wobei R₇ eine gesättigte oder ungesättigte, geradkettige oder verzweigte C₁₋₃₅-Kohlenwasserstoffgruppe ist, die gegebenenfalls eine oder mehrere Hydroxygruppen aufweist, wobei die Hydroxygruppe(n) der Gruppe R₇ mit einer gesättigten oder ungesättigten, geradkettigen oder verzweigten, gegebenenfalls ein- oder mehrfach hydroxylierten C₁₋₃₅-Fettsäure verestert sein kann (können);
oder eine Gruppe R"-(NR-CO)-R', wobei R Wasserstoff oder eine C₁₋₂₀-Kohlenwasserstoffgruppe mit einer oder mehreren Hydroxygruppen und vorzugsweise einer Hydroxygruppe bedeutet und R' und R" Kohlenwasserstoffgruppen sind, deren Summe an Kohlenstoffatomen im Bereich von 9 bis 30 liegt, wobei R' eine zweiwertige Gruppe ist,
oder eine Gruppe R₈-O-CO-(CH₂)ₚ, wobei R₈ eine C₁₋₂₀-Kohlenwasserstoffgruppe ist und p eine ganze Zahl im Bereich von 1 bis 12 bedeutet;
- R₂ eine Gruppe, die ausgewählt ist unter Wasserstoff, einer Gruppe vom Saccharidtyp, insbesondere (Glycosyl)ₙ, (Galactosyl)ₘ oder Sulfogalactosyl, einer Sulfat- oder Phosphatgruppe, einer Phosphorylethylamingruppe und einer Phosphorylethylammoniumgruppe, wobei n eine ganze Zahl im Bereich von 1 bis 4 und m eine ganze Zahl im Bereich von 1 bis 8 ist;
- R₃ Wasserstoff oder eine gesättigte oder ungesättigte, gegebenenfalls hydroxylierte C₁₋₃₃-Kohlenwasserstoffgruppe, wobei die Hydroxygruppe(n) mit einer anorganischen Säure oder einer Säure R₇COOH verestert, wobei R₇ die oben angegebenen Bedeutungen aufweist, oder mit einer (Glycosyl)ₙ-Gruppe, (Galactosyl)ₘ-Gruppe, Sulfogalactosylgruppe, Phosphorylethylamingruppe oder einer Phosphorylethylammoniumgruppe verethert sein kann (können), und wobei die Gruppe R₃ mit einer oder mehreren C₁₋₁₄-Alkylgruppen substituiert sein kann; R₃ bedeutet vorzugsweise eine α-Hydroxy-alkylgruppe mit 15 bis 26 Kohlenstoffatomen, wobei die Hydroxygruppe gegebenenfalls mit einer α-Hydroxysäure mit 16 bis 30 Kohlenstoffatomen verestert sein kann;
- R₄ Wasserstoff, Methyl, Ethyl, eine gesättigte oder ungesättigte, geradkettige oder verzweigte, gegebenenfalls hydroxylierte C₃₋₅₀-Kohlenwasserstoffgruppe, eine Gruppe -CH₂-CHOH-CH₂-O-R₆, wobei R₆ eine C₁₀₋₂₆-Kohlenwasserstoffgruppe bedeutet, oder eine Gruppe R₈-O-CO-(CH₂)ₚ, wobei R₈ eine C₁₋₂₀-Kohlenwasserstoffgruppe bedeutet und p eine ganze Zahl im Bereich von 1 bis 12 ist;
- R₅ Wasserstoff oder eine gesättigte oder ungesättigte, geradkettige oder verzweigte, gegebenenfalls ein- oder mehrfach hydroxylierte C₁₋₃₀-Kohlenwasserstoffgruppe, wobei die Hydroxygruppe(n) mit einer (Glycosyl)ₙ-Gruppe, (Galactosyl)ₘ-Gruppe, Sulfogalactosylgruppe, Phosphorylethylamingruppe und einer Phosphorylethylammoniumgruppe verethert sein kann (können);
mit der Maßgabe, daß R₄ nicht Wasserstoff, Methyl oder Ethyl bedeutet, wenn R₃ und R₅ Wasserstoff bedeuten oder wenn R₃ Wasserstoff und R₅ Methyl bedeutet.

2. Zusammensetzung nach Anspruch 1, **dadurch gekennzeichnet, daß** das UV-Filter eine Verbindung mit 3-Benzyliden-2-campher-struktur ist.

3. Zusammensetzung nach Anspruch 2, **dadurch gekennzeichnet, daß** das UV-Filter einer der folgenden Formeln (I) bis (V) entspricht: 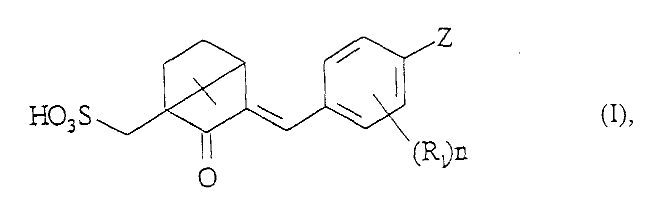 worin bedeuten:
- Z die Gruppe : 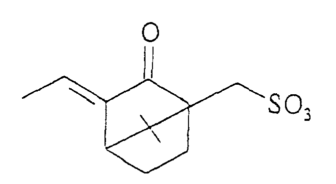
- n Null oder eine ganze Zahl im Bereich von 1 bis 4 (0 ≤ n < 4), und
- R₁ eine oder mehrere geradkettige oder verzweigte Alkyl- oder Alkoxygruppen mit 1 bis 4 Kohlenstoffatomen, die identisch oder voneinander verschieden sind; 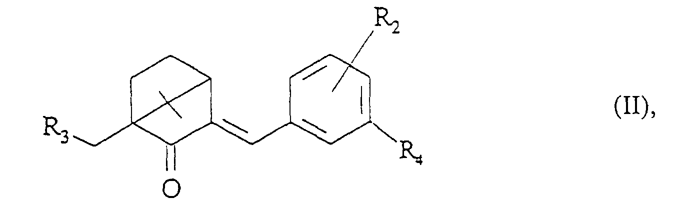
worin bedeuten:
- R₂ Wasserstoff, Halogen, eine Alkylgruppe mit 1 bis 4 Kohlenstoffatomen oder die Gruppe -SO₃H,
- R₃ und R₄ Wasserstoff oder die Gruppe -SO₃H, wobei mindestens eine der Gruppen R₂, R₃ oder R₄ die Gruppe -SO₃H bedeutet; 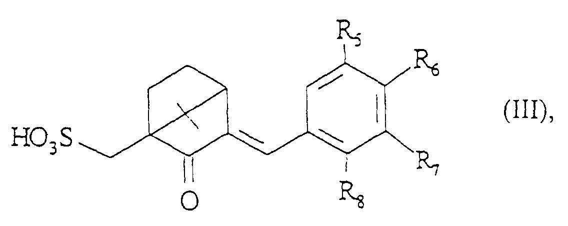
worin bedeuten:
- R₅ und R₇, die identisch oder voneinander verschieden sind, Wasserstoff, Hydroxy, eine geradkettige oder verzweigte Alkylgruppe mit 1 bis 8 Kohlenstoffatomen oder eine geradkettige oder verzweigte Alkoxygruppe mit 1 bis 8 Kohlenstoffatomen, wobei mindestens eine der Gruppen R₅ und R₇ Hydroxy, Alkyl oder Alkoxy bedeutet;
- R₆ und R₈, die identisch oder voneinander verschieden sind, Wasserstoff oder Hydroxy, wobei mindestens eine der Gruppen R₆ und R₈ Hydroxy bedeutet; 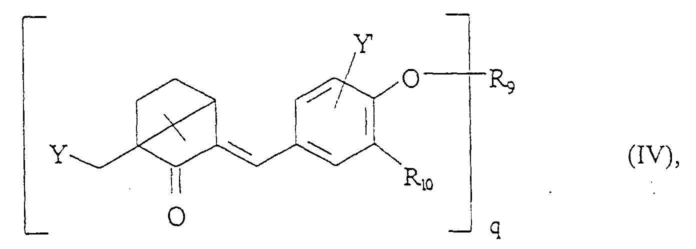
worin bedeuten:
- R₉ Wasserstoff, eine geradkettige oder verzweigte Alkylgruppe mit 1 bis 18 Kohlenstoffatomen, eine geradkettige oder verzweigte Alkenylgruppe mit 3 bis 18 Kohlenstoffatomen, die Gruppen 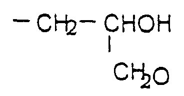 -(CH₂CH₂O)ₙ-H, -CH₂-CHOH-CH₃ oder die zweiwertigen Gruppen -(CH₂)ₘ- oder -CH₂-CHOH-CH₂-, wobei n eine ganze Zahl im Bereich von 1 bis 6 (1 ≤ n ≤ 6) und m eine ganze Zahl im Bereich von 1 bis 10 (1 ≤ m < 10) bedeutet,
- R₁₀ Wasserstoff, eine Alkoxygruppe mit 1 bis 4 Kohlenstoffatomen oder die zweiwertige Gruppe -O-, die an die Gruppe R₉ gebunden ist, wenn diese ebenfalls zweiwertig vorliegt,
- q 1 oder 2, mit der Maßgabe, daß R₉ eine zweiwertige Gruppe ist, wenn q 2 bedeutet,
- Y und Y' Wasserstoff oder die Gruppe -SO₃H, wobei mindestens eine der Gruppen Y und Y' von Wasserstoff verschieden ist; 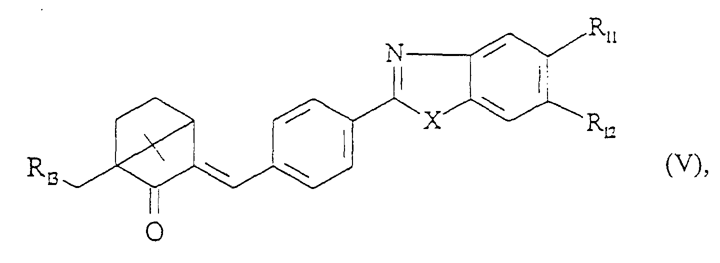
worin bedeuten:
- R₁₁ Wasserstoff, eine geradkettige oder verzweigte Alkyl- oder Alkoxygruppe mit 1 bis 6 Kohlenstoffatomen oder die Gruppe -SO₃H,
- R₁₂ Wasserstoff oder eine geradkettige oder verzweigte Alkyl- oder Alkoxygruppe mit 1 bis 6 Kohlenstoffatomen,
- R₁₃ Wasserstoff oder die Gruppe -SO₃H,
- wobei mindestens eine der Gruppen R₁₁ oder R₁₃ eine Gruppe -SO₃H bedeutet,
- X ein Sauerstoffatom oder Schwefelatom oder eine Gruppe -NR-, wobei R Wasserstoff oder eine geradkettige oder verzweigte Alkylgruppe mit 1 bis 6 Kohlenstoffatomen bedeutet.

4. Zusammensetzung nach Anspruch 3, **dadurch gekennzeichnet, daß** es sich bei der Verbindung der Formel (I) um Benzol-1,4-[di(3-methylidencampher-10-sulfonsäure)] handelt, die Verbindungen der Formel (II) unter 4-(3-Methylidencampher)benzolsul fonsäure, 3-Benzylidencampher-10-sulfonsäure, 2-Methyl-5-(3-methylidencampher)-benzolsulfonsäure, 2-Chlor-5-(3-methylidencampher)-benzolsulfonsäure und 3-(4-Methyl)-benzylidencampher-10-sulfonsäure ausgewählt sind, die Verbindungen der Formel (III) unter (3-*tert*.-Butyl-2-hydroxy-5-methyl)-benzylidencampher-10-sulfonsäure, (3*-tert.*-Butyl-2-hydroxy-5-methoxy)-benzylidencampher-10-sulfonsäure und (3,5-Di*-tert.*-butyl-4-hydroxy)-benzylidencampher-10-sulfonsäure ausgewählt sind, die Verbindungen der Formel (IV) unter 2-Methoxy-5-(3-methylidencampher)-benzolsulfonsäure, 3-(4,5-Methylendioxy)-benzyliden-campher-10-sulfonsäure, 3-(4-Methoxy)-benzyliden-campher-10-sulfonsäure, 3-(4,5-Dimethoxy)-benzylidencampher-10-sulfonsäure, 3-(4-n-Butoxy)-benzylidencampher-10-sulfonsäure und 3-(4-n-Butoxy-5-methoxy)-benzylidencampher-10-sulfonsäure ausgewählt sind und die Verbindung (V) die 2-[4-(Camphomethyliden)phenyl]benzimidazol-5-sulfonsäure ist.

5. Zusammensetzung nach Anspruch 1, **dadurch gekennzeichnet, daß** das UV-Filter eine Verbindung der Formel (VI) ist: 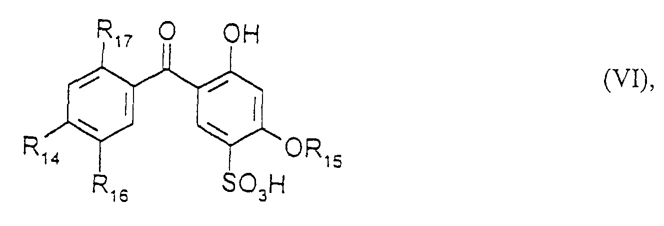 worin bedeuten:
- R₁₄ Wasserstoff oder eine geradkettige oder verzweigte Alkoxygruppe mit 1 bis 8 Kohlenstoffatomen,
- R₁₅ Wasserstoff oder eine geradkettige oder verzweigte Alkylgruppe mit 1 bis 8 Kohlenstoffatomen,
- R₁₆ Wasserstoff oder -SO₃H, und
- R₁₇ Wasserstoff oder Hydroxy.

6. Zusammensetzung nach Anspruch 5, **dadurch gekennzeichnet, daß** die Verbindung der Formel (VI) die 2-Hydroxy-4-methoxybenzophenon-5-sulfonsäure ist.

7. Zusammensetzung nach Anspruch 1, **dadurch gekennzeichnet, daß** das UV-Filter eine Verbindung der Formel (VII) ist: 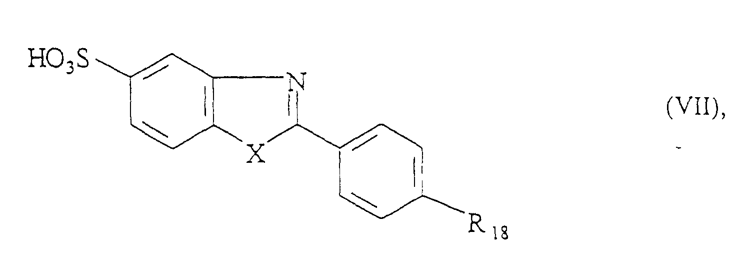 worin bedeuten:
- X ein Sauerstoffatom oder die Gruppe -NH-, und
- R₁₈ Wasserstoff, eine geradkettige oder verzweigte Alkyl- oder Alkoxygruppe mit 1 bis 8 Kohlenstoffatomen oder eine Gruppe der Formel (VIII): 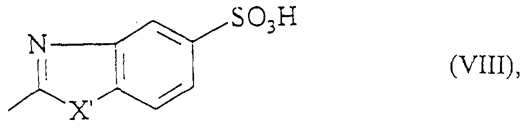
worin X' unabhängig von X ein Sauerstoffatom oder die Gruppe -NH- bedeutet.

8. Zusammensetzung nach Anspruch 7, **dadurch gekennzeichnet, daß** die Verbindung der Formel (VII) unter 2-Phenylbenzimidazol-5-sulfonsäure, Benzol-1,4-di(benzimidazol-2-yl-5-sulfonsäure) und Benzol-1,4-di(benzoxazol-2-yl-5- 5-sulfonsäure) ausgewählt ist.

9. Zusammensetzung nach einem der Ansprüche 1 bis 8, **dadurch gekennzeichnet, daß** die Verbindung vom Ceramidtyp unter den folgenden Verbindungen und ihren Gemischen ausgewählt ist:
- 2-N-Linoleoylamino-octadecan-1,3-diol,
- 2-N-Oleoylamino-octadecan-1,3-diol,
- 2-N-Palmitoylamino-octadecan-1,3-diol,
- 2-N-Stearoylamino-octadecan-1,3-diol,
- 2-N-Behenoylamino-octadecan-1,3-diol
- 2-N-[2-Hydroxy-palmitoyl]-amino-octadecan-1,3-diol,
- 2-N-Stearoylamino-octadecan-1,3,4-triol, und
- 2-N-Palmitoylamino-hexadecan-1,3-diol.

10. Zusammensetzung nach einem der Ansprüche 1 bis 8, **dadurch gekennzeichnet, daß** Verbindung vom Ceramidtyp unter (Bis(N-hydroxyethyl-N-cetyl)-malonamid), N-(2-Hydroxyethyl)-N-(3-cetyloxy-2-hydroxypropyl)-cetylsäureamid und N-Docosanoyl-N-methyl-D-glucamin ausgewählt ist.

11. Zusammensetzung nach einem der vorhergehenden Ansprüche, **dadurch gekennzeichnet, daß** sie 0,05 bis 10 Gew.-% und vorzugsweise 0,1 bis 5 Gew.-% UV-Filter, bezogen auf das Gesamtgewicht der Zusammensetzung, enthält.

12. Verwendung von Zusammensetzungen nach einem der Ansprüche 1 bis 11 als kosmetische Zusammensetzungen oder zur Herstellung von kosmetischen Zusammensetzungen zum Schutz der Haare gegen UV-Strahlung und insbesondere gegen Sonnenlicht.

13. Verfahren zur kosmetischen Behandlung zum Schutz der Haare gegen UV-Strahlung und insbesondere gegen Sonnenlicht, **dadurch gekennzeichnet, daß** es darin besteht, auf die Haare eine wirksame Menge einer Zusammensetzung nach einem der Ansprüche 1 bis 11 aufzutragen.

14. Verwendung einer Verbindung vom Ceramidtyp in einer kosmetischen Zusammensetzung für das Haar, die mindestens ein hydrophiles UV-Filter mit mindestens einer Sulfonsäuregruppe enthält, zur Verbesserung der Fixierung des UV-Filters auf dem Haar.

## Claims

1. Cosmetic hair composition, **characterized in that** it comprises, in a cosmetically acceptable medium, at least one ceramide-type compound and at least one agent for screening out ultraviolet radiation, containing at least one sulphonic acid radical;
the ceramide-type compound corresponding to the general formula (IX): 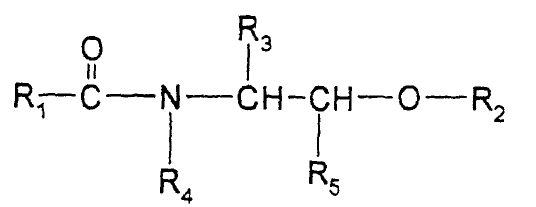 in which:
- R₁ denotes:
- either a linear or branched, saturated or unsaturated C₁-C₅₀, preferably C₅-C₅₀, hydrocarbon radical, it being possible for this radical to be substituted with one or more hydroxyl groups, optionally esterified with an acid R₇COOH, R₇ being a saturated or unsaturated, linear or branched, optionally mono- or polyhydroxylated C₁-C₃₅ hydrocarbon radical, it being possible for the hydroxyl (s) of the radical R₇ to be esterified with a saturated or unsaturated, linear or branched, optionally mono- or polyhydroxylated C₁-C₃₅ fatty acid;
- or a radical R"-(NR-CO)-R', R denotes a hydrogen atom or a mono- or polyhydroxylated, preferably monohydroxylated, C₁-C₂₀ hydrocarbon radical, R' and R" are hydrocarbon radicals in which the sum of the carbon atoms is between 9 and 30, R' being a divalent radical;
- or a radical R₈-O-CO-(CH₂)ₚ, R₈ denotes a C₁-C₂₀ hydrocarbon radical and p is an integer ranging from 1 to 12;
- R₂ is chosen from a hydrogen atom, a saccharide-type radical, in particular a (glycosyl)ₙ, (galactosyl)ₘ or sulphogalactosyl radical, a sulphate or phosphate residue, a phosphorylethylamine radical and a phosphorylethylammonium radical, in which n is an integer ranging from 1 to 4 and m is an integer ranging from 1 to 8;
- R₃ denotes a hydrogen atom or a saturated or unsaturated, hydroxylated or non-hydroxylated C₁-C₃₃ hydrocarbon radical, it being possible for the hydroxyl (s) to be esterified with an inorganic acid or an acid R₇COOH, R₇ having the same meanings as above, it being possible for the hydroxyl (s) to be etherified with a (glycosyl)ₙ, (galactosyl)ₘ, sulphogalactosyl, phosphorylethylamine or phosphorylethylammonium radical, it being possible for R₃ also to be substituted with one or more C₁-C₁₄ alkyl radicals;
preferably, R₃ denotes a C₁₅-C₂₆ α-hydroxyalkyl radical, the hydroxyl group optionally being esterified with a C₁₆-C₃₀ α-hydroxy acid;
- R₄ denotes a hydrogen atom, a methyl or ethyl radical, a saturated or unsaturated, linear or branched, optionally hydroxylated C₃-C₅₀ hydrocarbon radical or a radical -CH₂-CHOH-CH₂-O-R₆ in which R₆ denotes a C₁₀-C₂₆ hydrocarbon radical or a radical R₈-O-CO-(CH₂)ₚ, R₈ denotes a C₁-C₂₀ hydrocarbon radical and p is an integer ranging from 1 to 12,
- R₅ denotes a hydrogen atom or a saturated or unsaturated, linear or branched, optionally mono- or polyhydroxylated C₁-C₃₀ hydrocarbon radical, it being possible for the hydroxyl(s) to be etherified with a (glycosyl)ₙ, (galactosyl)ₘ, sulphogalactosyl, phosphorylethylamine or phosphorylethylammonium radical,
with the proviso that when R₃ and R₅ denote hydrogen or when R₃ denotes hydrogen and R₅ denotes methyl, then R₄ does not denote a hydrogen atom or a methyl or ethyl radical.

2. Composition according to Claim 1, **characterized in that** the agent for screening out ultraviolet radiation is a compound of 3-benzylidene-2-camphor structure.

3. Composition according to Claim 2, **characterized in that** the agent for screening out ultraviolet radiation corresponds to one of formulae (I) to (V) below: 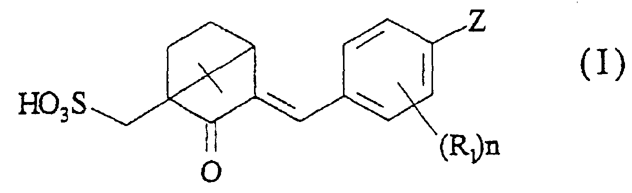 in which:
- Z denotes a group: 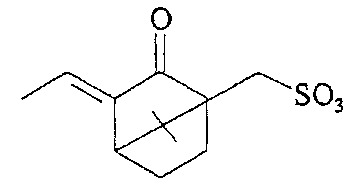
- n denotes 0 or an integer greater than or equal to 1 and less than or equal to 4,
- R₁ represents one or more identical or different, linear or branched alkyl or alkoxy radicals containing from 1 to 4 carbon atoms. 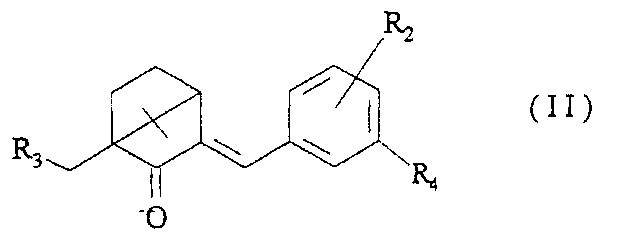
in which
- R₂ denotes a hydrogen atom, a halogen atom, an alkyl radical containing 1 to 4 carbon atoms or an -SO₃H radical,
- R₃ and R₄ denote a hydrogen atom or an -SO₃H radical, at least one of the radicals R₂, R₃ and R₄ denoting the -SO₃H radical. 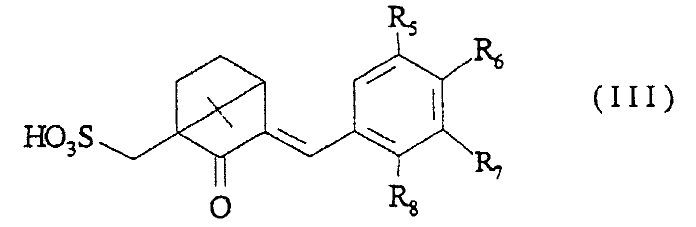
in which:
- R₅ and R₇, which may be identical or different, denote a hydrogen atom, a hydroxyl radical, a linear or branched alkyl radical containing 1 to 8 carbon atoms or a linear or branched alkoxy radical containing 1 to 8 carbon atoms, at least one of the radicals R₅ and R₇ representing a hydroxyl, alkyl or alkoxy radical,
- R₆ and R₈, which may be identical or different, denote a hydrogen atom or a hydroxyl radical, at least one of the radicals R₆ and R₈ denoting a hydroxyl radical. 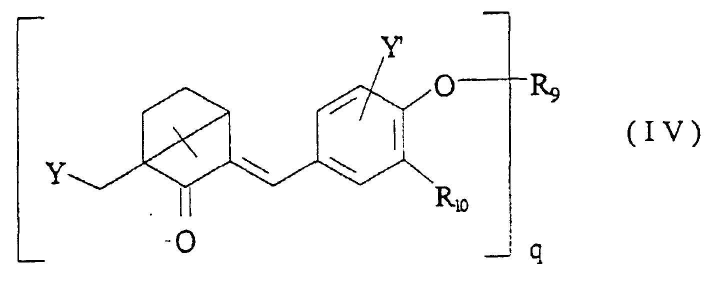
in which
- R₉ denotes a hydrogen atom, a linear or branched alkyl radical containing from 1 to 18 carbon atoms, a linear or branched alkenyl radical containing from 3 to 18 carbon atoms, a group chosen from: 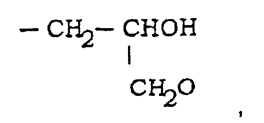 or -(CH₂CH₂O)ₙ-H, or -CH₂-CHOH-CH₃,
or -(CH₂)ₘ- or -CH₂-CHOH-CH₂-, n being an integer between 1 and 6 (1 ≤ n ≤ 6) and m being an integer between 1 and 10 (1 ≤ m ≤ 10),
- R₁₀ denotes a hydrogen atom, an alkoxy radical containing from 1 to 4 carbon atoms or a divalent radical -O- connected to the radical R₉ when the latter is also divalent,
- q denotes an integer equal to 1 or 2, it being understood that if q is equal to 2, R₉ must denote a divalent radical,
- Y and Y' denote a hydrogen atom or an -SO₃H radical, at least one of these radicals Y and Y' being other than hydrogen. 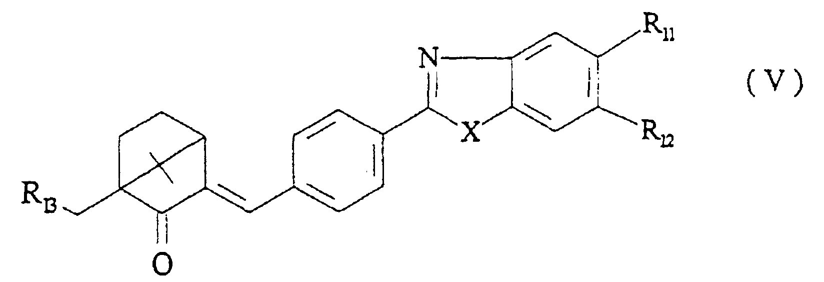
in which:
- R₁₁ denotes a hydrogen atom, a linear or branched alkyl or alkoxy radical containing 1 to 6 carbon atoms or an -SO₃H radical,
- R₁₂ denotes a hydrogen atom or a linear or branched alkyl or alkoxy radical containing 1 to 6 carbon atoms,
- R₁₃ denotes a hydrogen atom or an -SO₃H radical,
- at least one of the radicals R₁₁ and R₁₃ denotes an
- SO₃H radical,
- X is an oxygen or sulphur atom or a group -NR-, R being a hydrogen atom or a linear or branched alkyl radical containing from 1 to 6 carbon atoms.

4. Composition according to Claim 3, **characterized in that** the compound of formula (I) is benzene-1,4-[di-(3-methylidenecamphor-10-sulphonic acid)], compounds of formula (II) are chosen from 4-(3-methylidenecamphor)benzenesulphonic acid, 3-benzylidenecamphor10-sulphonic acid, 2-methyl-5-(3-methylidenecamphor)benzenesulphonic acid, 2-chloro-5-(3-methylidenecamphor)benzenesulphonic acid, 3-(4-methyl)benzylidenecamphor-10-sulphonic acid, the compounds of formula (III) are chosen from (3-t-butyl-2-hydroxy-5-methyl)benzylidenecamphor-10-sulphonic acid, (3-t-butyl-2-hydroxy-5-methoxy)benzylidenecamphor-10-sulphonic acid, (3,5-di-tert-butyl-4-hydroxy)benzylidenecamphor-10-sulphonic acid, the compounds of formula (IV) are chosen from 2-methoxy-5-(3-methylidenecamphor)benzenesulphonic acid, 3-(4,5-methylenedioxy)benzylidenecamphor-10-sulphonic acid, 3-(4-methoxy)benzylidenecamphor-10-sulphonic acid, 3-(4,5-dimethoxy)benzylidenecamphor-10-sulphonic acid, 3-(4-n-butoxy)benzylidenecamphor-10-sulphonic acid, 3-(4-n-butoxy5-methoxy)benzylidenecamphor-10-sulphonic acid, the compound of formula (V) is 2-[4-(camphormethylidene)phenyl]benzimidazole-5-sulphonic acid.

5. Composition according to Claim 1, **characterized in that** the agent for screening out ultraviolet radiation is a compound of formula (VI): 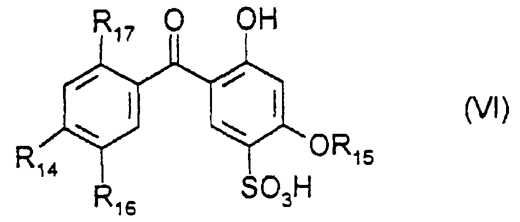 in which:
- R₁₄ denotes either a hydrogen atom or a linear or branched alkoxy radical containing from 1 to 8 carbon atoms,
- R₁₅ denotes either a hydrogen atom or a linear or branched alkyl radical containing from 1 to 8 carbon atoms,
- R₁₆ denotes either a hydrogen atom or an SO₃H group,
- R₁₇ denotes either a hydrogen atom or a hydroxyl radical.

6. Composition according to Claim 5, **characterized in that** the compound of formula (VI) is 2-hydroxy-4-methoxybenzophenone-5-sulphonic acid.

7. Composition according to Claim 1, **characterized in that** the agent for screening out ultraviolet radiation is a compound of formula (VII): 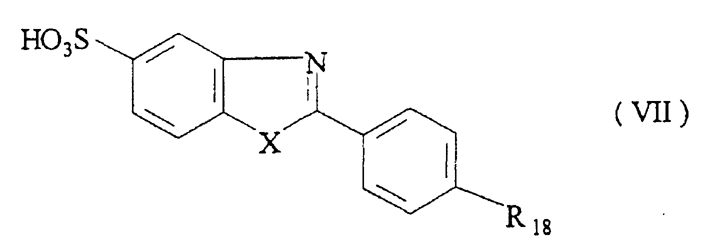 in which:
- X denotes an oxygen atom or an -NH- radical,
- R₁₈ denotes a hydrogen atom, a linear or branched alkyl or alkoxy radical containing from 1 to 8 carbon atoms or a group of formula (VIII) 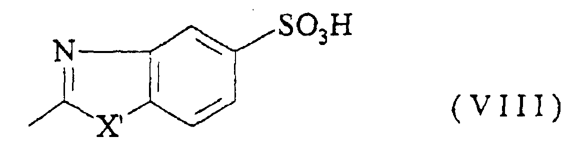 in which X' denotes, independently of X, an oxygen atom or an -NH- radical.

8. Composition according to Claim 7, **characterized in that** the compound of formula (VII) is chosen from 2-phenylbenzimidazole-5-sulphonic acid, benzene-1,4-di(benzimidazol-2-yl-5-sulphonic acid) and benzene-1,4-di(benzoxazol-2-yl-5-sulphonic acid).

9. Composition according to any one of Claims 1 to 8, **characterized in that** the ceramide-type compound is chosen from the group consisting of:
- 2-N-linoleoylaminooctadecane-1,3-diol,
- 2-N-oleoylaminooctadecane-1,3-diol,
- 2-N-palmitoylaminooctadecane-1,3-diol,
- 2-N-stearoylaminooctadecane-1,3-diol,
- 2-N-behenoylaminooctadecane-1,3-diol,
- 2-N-[2-hydroxypalmitoyl]aminooctadecane-1,3-diol,
- 2-N-stearoylaminooctadecane-1,3,4 triol,
- 2-N-palmitoylaminohexadecane-1,3-diol,
or mixtures of these compounds.

10. Composition according to any one of Claims 1 to 8, **characterized in that** the ceramide-type compound is chosen from bis(N-hydroxyethyl-N-cetyl)malonamide, cetylic acid N-(2-hydroxyethyl)-N-(3-cetyloxy-2-hydroxypropyl)amide and N-docosanoyl-N-methyl-D-glucamine.

11. Composition according to any one of the preceding claims, **characterized in that** it contains from 0.05 to 10% by weight of agent for screening out ultraviolet radiation, and preferably 0.1 to 5% by weight, relative to the total weight of the composition.

12. Use of the compositions defined in any one of Claims 1 to 11 as, or for the manufacture of, cosmetic compositions for protecting the hair against ultraviolet radiation, in particular solar radiation.

13. Cosmetic treatment process for protecting the hair against ultraviolet radiation, in particular solar radiation, **characterized in that** it consists in applying to the hair an effective amount of a composition as defined in any one of Claims 1 to 11.

14. Use of a ceramide-type compound in a cosmetic hair composition containing a hydrophilic UV screening agent containing at least one sulphonic acid radical, in order to improve the fixing of the said UV screening agent to the hair.
